# EUROPEAN PATENT APPLICATION

(11) **EP 1 792 896 A1**
(43) Date of publication of application: **06.06.2007**
(21) Application number: 05026160.1
(22) Date of filing: 01.12.2005
(51) Int. Cl.: C07D 209/42, C07C 227/40, C07C 227/42

(54) **Process for the preparation of perindopril and salts thereof**

(71) Applicant: KRKA, tovarna zdravil, d.d., Novo mesto, 8501 Novo mesto (SI)
(72) Inventor: Weiren, Chen, Jiangdong Nigbo (CN); Jianxiang, Shi, Shanghai (CN); Biao, Lu, Guangde (CN)
(74) Representative: UEXKÜLL & STOLBERG

(57) **Abstract**

The present invention relates to a process for the preparation of the ACE inhibitor (2S,3aS,7aS)-1-((2S)-2-(((1S)-1-(ethoxycarbonyl)butyl)amino)-1-oxopropyl)octahydro-1H-indol-2-carboxylic acid and of pharmaceutically acceptable salts thereof as well as to processes for preparing a N-((S)-1-carbethoxybutyl)-(S)-alanine intermediate and a (2S,3aS,7aS)-octahydroindole-2-carboxylic acid intermediate in a purified form.

## Description

This invention relates to a process for the preparation of perindopril and salts thereof as well as to processes for purifying intermediates used in this preparation.

Perindopril is the generic name of (2S,3aS,7aS)-1-((2S)-2-(((1S)-1-(ethoxycarbonyl)butyl)amino)-1-oxopropyl)octahydro-1*H*-indol-2-carboxylic acid. Perindopril and its salts, preferably the *t*-butylamine salt thereof, referred to as perindopril erbumine, are compounds having ACE inhibitory action and are therefore useful for the treatment of hypertension.

Commonly, perindopril is prepared by coupling N-((S)-1-carbethoxybutyl)-(S)-alanine or an activated derivative thereof with an ester and/or salt of (2S,3aS,7aS)-octahydroindole-2-carboxylic acid.

For example, EP 308 341 teaches a large-scale synthesis of perindopril in the form of the *t*-butylamine salt. (2S,3aS,7aS)-octahydroindole-2-carboxylic acid benzyl ester p-toluenesulfonic salt and N-((S)-1-carbethoxybutyl)-(S)-alanine are reacted in the presence of triethyl amine, N,N-dicyclohexylcarbodiimide and 1-hydroxybenztriazole. After completion of the reaction, perindopril benzyl ester is obtained, which is reduced, lyophilized and then converted into a salt with *t*-butylamine in ethyl acetate.

Vincent et al. describe in Tetrahydron Letters, Vol. 23, 16, 1677-1680 that the alanine intermediate N-((S)-1-carbethoxybutyl)-(S)-alanine is prepared by catalytic reductive amination of pyruvic acid and (S)-norvaline ethyl ester hydrochloride in ethanol.

EP 308 340 teaches to condense (S)-norvaline ethyl ester hydrochloride with pyruvic acid in an aqueous medium to give a high proportion of the diastereomer of the alanine intermediate in which both asymmetric carbon atoms have the (S)-configuration and to isolate the obtained diastereomer by one single crystallization from a polar organic solvent carefully selected from acetonitrile, ethyl acetate and lower aliphatic alcohol alone or mixed with each other or with water.

Furthermore, WO 01/56353 discloses a method for the industrial synthesis of the alanine intermediate, wherein sodium pyruvate is condensed with (S)-norvaline ethyl ester hydrochloride by hydrogenation in water in the presence of a specific amount of sodium hydroxide. After hydrogenation the reaction mixture is acidified and filtered to give directly the alanine intermediate.

For the synthesis of the (2S,3aS,7aS)-octahydroindole-2-carboxylic acid intermediate, Vincent et al. teach in Tetrahydron Letters, Vol. 23, 16, 1677-1680 to convert (S)-indoline-2-carboxylic acid into its hydrochloride salt which is subsequently catalytically hydrogenated using Pd/C. The resulting ethyl ester hydrochloride salt is then saponified and hydrogenated to produce the (2S,3aS,7aS)-octahydroindole-2-carboxylic acid intermediate.

In EP 308 339 a synthesis of the indole intermediate is described wherein (S)-indoline-2-carboxylic acid is hydrogenated in the presence of supported Ni, Pt, Pd or Rh catalyst and the desired (2S,3aS,7aS)-isomer is separated from the less obtained (2S,3aR,7aR)-isomer by a single crystallization from specific polar solvents.

WO 2005/100317 describes a simplified process step for preparing (2S,3aS,7aS)-octahydroindole-2-carboxylic acid by hydrogenation of (S)-indoline-2-carboxylic acid under reduced pressure conditions in an alkaline medium including a recrystallization of the intermediate from acetonitrile. Subsequently, a substituted benzyl ester of (2S,3aS,7aS)-octahydroindole-2-carboxylic acid is coupled with the alanine intermediate to give perindopril which is converted into perindopril erbumine.

However, the known methods for preparing perindopril have not turned out to be satisfactory in respect of the purity obtained.

Therefore, there still exists a need for an improved process for perindopril synthesis which can also be used on an industrial scale, whereby a high purity grade of the final product without substantial amounts of undesired by-products can be achieved.

This object is surprisingly solved by the process for the preparation of perindopril according to claims 1 to 15.

The invention is also directed to a process for preparing N-((S)-1-carbethoxybutyl)-(S)-alanine, or an activated or protected form or a salt thereof, in purified form according to claims 16 and 17 and a process for preparing (2S,3aS,7aS)-octahydroindole-2-carboxylic acid, or an activated or protected form or a salt thereof, according to claims 18 and 19.

The process according to the invention for the preparation of perindopril or of pharmaceutically acceptable salts of perindopril is characterized in that
(i) N-((S)-1-carbethoxybutyl)-(S)-alanine or an activated or protected form or salt thereof
   and/or
(ii) (2S,3aS,7aS)-octahydroindole-2-carboxylic acid or an activated or protected form or salt thereof
   is used in a purified form as intermediate in the preparation of perindopril, wherein the purified form has been prepared by using
(iii) a solvent selected from the group consisting of tetrahydrofuran, dimethyl carbonate, diethyl carbonate, 1,2-dimethoxy ethane, tert.-butyl methyl ether, 2-methyl tetrahydrofuran, cyclopentyl methyl ether and mixtures thereof,
or a mixture of solvent (iii) and water.

A particularly preferred pharmaceutically acceptable salt of perindopril is the tert.-butylamine salt, i.e. perindopril erbumine.

In the process of the present invention N-((S)-1-carbethoxybutyl)-(S)-alanine or an activated form or a protected form or a salt thereof can be used as an intermediate in the synthesis of perindopril (intermediate (i)). The term "activated form" is intended to include derivatives of N-((S)-1-carbethoxybutyl)-(S)-alanine which have been formed by reacting with commonly used coupling agents known in the art such as N,N-thionyldiimidazole, N,N-dicyclohexylcarbodiimide and 1-hydroxybenzotriazole. These agents may convert the acid group of the alanine to groups which are more reactive in peptid coupling reactions, e.g. an acid chloride. Moreover, the term "protected form" is intended to include derivatives of N-((S)-1-carbethoxybutyl)-(S)-alanine in which functional groups and in particular the amine group of the alanine has been protected with usual protecting groups such as acyl, silyl groups, and carbamides. Finally, also salts of N-((S)-1-carbethoxybutyl)-(S)-alanine such as the hydrochloride salt can be used as intermediate (i).

Alternatively to or in combination with such an alanine derivative, (2S,3aS,7aS)-octahydroindole-2-carboxylic acid or an activated form or a protected form or a salt thereof can be used as an intermediate in the present process (intermediate (ii)). The term "activated form" is intended to include derivatives of (2S,3aS,7aS)-octahydroindole-2-carboxylic acid which are formed upon reaction with commonly used coupling agents known in the art such as N,N-thionyldiimidazole, N,N-dicyclohexylcarbodiimide and 1-hydroxybenzotriazole. The term "protected form" is intended to include derivatives of (2S,3aS,7aS)-octahydroindole-2-carboxylic acid in which functional groups and in particular the acid group are protected by a protecting group such as benzyl, substituted benzyl, t-butyl or trimethylsilyl. The intermediate (ii) can also be a salt of (2S,3aS,7aS)-octahydroindole-2-carboxylic acid such as the potassium, lithium or sodium salt.

Publications relating to N-((S)-1-carbethoxybutyl)-(S)-alanine, (2S,3aS,7aS)-octahydroindole-2-carboxylic acid and their salts and activated and protected forms as well as their use in the preparation of perindopril are for example: EP 37 231, EP 308 339, EP 308 340, EP 308 341, EP 309 324, EP 937 714, EP 1 362 845, EP 1 400 531, WO 01/56353, WO 01/56972, WO 04/92095 and WO 2005/100317.

It has surprisingly been found that perindopril of high quality and purity is provided without substantial amounts of by-products by using intermediate (i) and/or intermediate (ii) in a purified form, wherein the purified form has been prepared by using solvent (iii) or a mixture of solvent (iii) and water. In particular, it has been found that both the chemical purity and the enantiomeric purity is increased by the method of the invention.

It is particularly preferred to use tetrahydrofuran (THF) as solvent (iii).

According to one aspect of the invention, a purified form of intermediate (i) is used. It is particularly preferred that intermediate (i) is N-((S)-1-carbethoxybutyl)-(S)-alanine.

Preferably, the purified form of intermediate (i) is prepared by a recrystallization from solvent (iii) or a mixture of solvent (iii) and water. Advantageously, THF is used as solvent (iii), i.e. the purified form of intermediate (i) is preferably prepared by a recrystallization from THF or a mixture of THF and water. It is also possible that the purified form of the alanine intermediate (i) is prepared by multiple recrystallizations such as 2 or 3 recrystallizations from solvent (iii) or a mixture of solvent (iii) and water.

Further, it is preferred that the recrystallization for preparing purified intermediate (i) such as purified N-((S)-1-carbethoxybutyl)-(S)-alanine comprises the steps of
(a) mixing crude intermediate (i) and solvent (iii) to give a suspension,
(b) optionally adding charcoal to the suspension of step (a),
(c) heating the suspension of step (a) or (b) to obtain a solution of intermediate (i), preferably heating to reflux,
(d) filtering the solution of step (c),
(e) optionally adding water to the filtrate of step (d),
(f) cooling the filtrate of step (d) or the mixture of step (e) to precipitate intermediate (i), and
(g) collecting the obtained intermediate (i).

It is particularly preferred that in step (f) the filtrate or mixture is cooled to a temperature of about -20 to about 30 °C, preferably of about 0 to about 25 °C and more prefarably of about 10 to about 25 °C.

Furthermore, if a mixture of solvent (iii) and water is used for the purification of the alanine intermediate (i), it is preferred that the ratio of solvent (iii) to water (by volume) ranges from 0.5:1 to 100:1, preferably 1:1 to 50:1 and more preferably 5:1 to 30:1. These mixing ratios of solvent (iii) and water can be achieved by initially mixing the solvents before contacting them with the alanine intermediate to be purified or by firstly mixing one of the solvents with the alanine intermediate and adding the other solvent at a later step of the purification procedure as it is for example provided in step (e) of the above described preferred recrystallization method.

It has been found that the use of a mixture of THF and water for purifying N-((S)-1-carbethoxybutyl)-(S)-alanine instead of THF alone prevents the formation of THF solvates of the alanine intermediate which solvates may be more difficult to be recovered in a pure form. Nevertheless, the sole use of THF also results in highly purified N-((S)-1-carbethoxybutyl)-(S)-alanine.

According to another aspect of the invention, a purified form of intermediate (ii) is used in the synthesis of perindopril. It is particularly preferred that intermediate (ii) is (2S,3aS,7aS)-octahydroindole-2-carboxylic acid.

In particular, also the purified form of the indole intermediate (ii) is prepared by a recrystallization from solvent (iii) or a mixture of solvent (iii) and water.

In particular, THF or 1,2-dimethoxy ethane are used as solvent (iii).

Preferably, a mixture of solvent (iii) and water is used for recrystallizing intermediate (ii). In addition, it is particularly preferred that the ratio by volume of solvent (iii) to water ranges from 0.5:1 to 50:1, preferably 1:1 to 20:1 1 and more preferably 2:1 to 8:1.

In one preferred embodiment, the recrystallization of the indole intermediate (ii) comprises
(a) heating a mixture of crude intermediate (ii), solvent (iii) and water to give a solution, preferably heating to reflux,
(b) optionally filtering the solution of step (a),
(c) cooling the solution of step (a) or the filtrate of step (b) to precipitate purified intermediate (ii), and
(d) collecting the obtained intermediate (ii).

In another embodiment of the present process, the indole intermediate (ii) is purified by a recrystallization comprising
(a') heating a mixture of crude intermediate (ii) and water to give a solution, preferably heating to a temperature from 60 to 75 °C,
(b') adding solvent (iii) to the solution of step (a),
(c') cooling the solution of step (b) to precipitate purified intermediate (ii), and
(d') collecting the obtained intermediate (ii).

For both embodiments of recrystallization, it is particularly preferred that in step (c) or (c') the solution of step (a), (a') or (b') or the filtrate of step (b) is cooled to a temperature of about -20 to about 30 °C, preferably to a temperature of about 0 to about 25 °C and more preferably to a temperature of about 10 to about 25 °C, in order to effect the precipitation of the purified indole intermediate.

It is even more advantageous if prior to step (d) or (d') the solution is kept at the precipitation temperature of step (c) or (c') for 0.5 to 6 h, preferably for 1 to 3 h.

Moreover, intermediate (ii) which has been purified according to the invention may be further washed with solvent (iii) or a mixture of solvent (iii) and water, and is particularly washed with solvent (iii). Preferably, for washing intermediate (ii) THF is used as solvent (iii).

As the use of solvent (iii) or a solvent (iii)/water mixture is particularly useful for purifying alanine intermediate (i), this invention further includes a process for preparing intermediate (i) selected from N-((S)-1-carbethoxybutyl)-(S)-alanine or an activated or protected form or a salt thereof in a purified form, wherein crude intermediate (i) is purified by using solvent (iii) or a mixture of solvent (iii) and water. Preferably, the purified form of the alanine intermediate (i) is prepared by a recrystallization from solvent (iii) or a mixture of solvent (iii) and water. It is particularly preferred to use THF as solvent (iii) for purifying the alanine intermediate (i).

Further preferred embodiments of the process for preparing intermediate (i) in a purified form can be taken from the above description of the present process for preparing perindopril, wherein a purified intermediate (i) is used.

Moreover, the invention also relates to a process for preparing intermediate (ii) selected from (2S,3aS,7aS)-octahydroindole-2-carboxylic acid or an activated or protected form or salt thereof in a purified form, wherein crude intermediate (ii) is purified by using solvent (iii) or a mixture of solvent (iii) and water. Advantageously, the purified form of the indole intermediate (ii) is prepared by a recrystallization from solvent (iii) or a mixture of solvent (iii) and water. Preferably, a mixture of solvent (iii) and water is used.

Further preferred embodiments of the process according to the invention for preparing intermediate (ii) and preferably (2S,3aS,7aS)-octahydroindole-2-carboxylic acid in a purified form are apparent from the description of the present perindopril synthesis process using purified intermediate (ii).

The present invention is further illustrated by the following

### Examples.

### Examples

### Example 1

### Step A: Preparation of N-((S)-1-carbethoxybutyl)-(S)-alanine

Ethyl L-norvalinate hydrochloride (80 g, 0.44 M) and 200 ml of ethanol (95 %) were charged into a hydrogenator. Pyruvic acid (40 g, 0.454 M) was added to the above mixture and the resulting mixture was stirred for 30 minutes. Then, 16 g of 10 % Pd/C were added to the mixture, the nitrogen atmosphere was replaced by hydrogen and the mixture was hydrogenated at 30°C and a pressure of 30 bar under stirring for 2 days. The catalyst was filtered off and the filtrate was neutralized with sodium hydroxide. The neutralized filtrate was concentrated to dryness under reduced pressure. The residue obtained after evaporation was suspended in ethanol. The suspension was filtered to remove any undissolved material and evaporated under reduced pressure. The crude residue was taken up in 560 ml of tetrahydrofurane (THF). Charcoal was added and the suspension was heated to its boiling point and filtered. The filtrate was then cooled to 20°C - 25°C. The precipitated solid was collected by filtration and dried. 36 g (yield: 37.6%) of N-((S)-1-carbethoxybutyl)-(S)-alanine as white solid were obtained.

This product could be employed in the further synthesis of perindopril without any additional treatment, in particular purification.

### Step B: Preparation of (2S,3aS,7aS)octahydro-1H-indol-2-carboxylic acid

Ethyl (S)-indoline-2-carboxylate hydrochloride (30 g, 0.13 M), 300 ml of ethanol and 3 g of 7.5 % Pd/C were charged under nitrogen into a hydrogenator. The nitrogen atmosphere was 3 times replaced with fresh nitrogen and 5 times with hydrogen. The reaction mixture was hydrogenated at 60°C and 50 bar under stirring until the hydrogen had been consumed. The catalyst was filtered off and 28 ml of sodium hydroxide (30 %) were added to the filtrate. The mixture was stirred at 30°C for 5 h. After hydrolysis, the pH was adjusted to 4.5 using 37% aqueous HCl. Sodium chloride was removed by filtration and the filtrate was concentrated under reduced pressure. 250 ml of ethanol were added to the residue, the mixture was heated to reflux and again sodium chloride was filtered off. The filtrate was concentrated under reduced pressure. 160 ml of tetrahydrofurane and 30 ml of water were added to the dry residue and the mixture was heated to reflux to obtain a clear solution. This solution was cooled to 10°C and aged at this temperature for 2 h. The precipitated crystals were filtered off, washed with 10 ml of tetrahydrofurane and dried at 70°C for 4 h. 9 g (yield: 40.4 %) of (2S,3aS,7aS)-octahydro-1*H*-indole-2-carboxylic acid as white powder were obtained. This product was employed in the next step without any additional treatment.

### Step C: Silylation of (2S,3aS,7aS)octahydro-1H-indol-2-carboxylic acid

Trimethyl chlorosilane (29.3 ml, 0.22M) and trietylamine (30.7 ml, 0.22 M) were added to a suspension of (2aS,3aS,7aS)-octahydro-1*H*-indole-2-carboxylic acid (18,6 g 0.11M) in 400 ml of dichloromethane. The mixture was stirred for 3 h at 20 to 25°C.

### Step D: Activation of N-((S)-1-carbethoxybutyl)-(S)-alanine with N,N-thionyldiimidazole

A solution of N-((S)-1-carbethoxybutyl)-(S)-alanine (21.7 g, 0.1 M) and imidazole (27.3 g, 0.4 M) in 400 ml of dichloromethane was cooled to -10°C and a solution of thionylchloride (7.6 ml, 0.115 M) was added. The stirring was continued for 2 h at the same temperature. After 2 h, imidazole hydrochloride was filtered off and the filtrate was used in the next step.

### Step E: Preparation of perindopril erbumin ((2S,3aS,7aS)-1-((2S)-2-(((1S)-1-(ethoxycarbonyl)butyl)amino)-1-oxopropyl)octahydro-1H-indol-2-carboxylic acid 2-methylpropane-2-amine salt)

To a cooled suspension (- 10 °C) of silylated (2S,3aS,7aS)-octahydro-1*H*-indole-2-carboxylic acid, the filtrate obtained as final product from step D and also cooled to -10°C was added. The reaction suspension was stirred for 3 h at -10°C and 18 h at 20-25°C. After the reaction was completed, 250 ml of water were added and the pH was adjusted to 5-6 using a 20 % aqueous solution of NaOH. The dichloromethane layer was separated and the water layer was extracted twice with each 150 ml of dichloromethane. The combined organic layers were concentrated under reduced pressure. The remaining oil was dissolved in 750 ml of acetonitrile and undissolved materials were filtered off. T-butylamine (11,1 ml, 0.1 M) was added to the filtrate. The suspension was heated to reflux and maintained at reflux until a clear solution was obtained. The clear solution was then cooled to 20 to 25°C and the precipitate was filtered and washed with acetonitrile. After drying at 40 - 45°C, perindopril erbumine was obtained (2,69 g yield: 61%).

### Example 2

### Preparation of N-((S)-1-carbethoxybutyl)-(S)-alanine

L-norvaline ethyl ester hydrochloride (50 g), 300 ml of ethanol and pyruvic acid sodium salt (31.8 g) were hydrogenated in the presence of 5 g of 7.5 % Pd/C (dry basis) for 40 h at 40 °C and at a pressure of 3 MPa. Then, the mixture was filtered to remove the catalyst and sodium chloride. The filtrate was evaporated to dryness. 300 ml of THF were added and the mixture was heated to reflux for 30 minutes and then filtered. 45 ml of water were added to the clear filtrate and the solution was cooled to 10 °C for crystallization. The obtained solid was collected and dried at 70 °C for 6 h to give 24 g of N-((S)-1-carbethoxybutyl)-(S)-alanine (HPLC: 92.4%; R-isomer, i.e. N-((S)-1-carbethoxybutyl)-(R)-alanine: 1.7%). This solid was again purified with THF and water as described above to obtain 19 g of the title compound (HPLC: 94.6%; R-isomer: 0.8%; optical rotation: +3.1°).

### Example 3

### Preparation of N-((S)-1-carbethoxybutyl)-(S)-alanine

L-norvaline ethyl ester hydrochloride (100 g), 400 ml of ethanol, 30 % aqueous sodium hydroxide solution (73.3 g) and pyruvic acid (51.7 g) were hydrogenated in the presence of 10 g of 7.5 % Pd/C (dry basis) for 10 h at 40 °C and at a pressure of 3 MPa. Then, the mixture was filtered to remove the catalyst and sodium chloride. The filtrate was evaporated to dryness. 700 ml of THF were added and the mixture was heated to reflux for 30 minutes and then filtered to remove sodium chloride. At a temperature of 60 °C, 30 ml of water were added to the clear filtrate and the solution was cooled to 10 °C for crystallization. The obtained solid was collected and dried to give 40 g of N-((S)-1-carbethoxybutyl)-(S)-alanine (HPLC: 97.7%; R-isomer: 0.8%, optical rotation: +2.6°).

### Example 4

### Preparation of N-((S)-1-carbethoxybutyl)-(S)-alanine

L-norvaline ethyl ester hydrochloride (50 g), 280 ml of 95 % ethanol and pyruvic acid sodium salt (31.8 g) were hydrogenated in the presence of 5 g of 7.5 % Pd/C (dry basis) for 10 h at 40 °C and at a pressure of 3 MPa. Then, the mixture was filtered to remove the catalyst and sodium chloride. The filtrate was evaporated to dryness. 350 ml of THF were added and the mixture was heated to reflux for 30 minutes and then filtered to remove sodium chloride. The filtrate was cooled to 10 °C for crystallization. The obtained solid was collected and dried to give 34 g of N-((S)-1-carbethoxybutyl)-(S)-alanine (HPLC: 91.4%; R-isomer: 1.8%). This solid was purified with THF again as described above to obtain 24 g of the title compound (HPLC: 97.6%; R-isomer: 0.48%; optical rotation: +2.78°).

### Example 5

### Preparation of N-((S)-1-carbethoxybutyl)-(S)-alanine

L-norvaline ethyl ester hydrochloride (50 g), 280 ml of 95 % ethanol, sodium hydroxide (11.3 g) and pyruvic acid (26 g) were hydrogenated in the presence of 5 g of 7.5 % Pd/C (dry basis) for 10 h at 40 °C and at a pressure of 3 MPa. Then, the mixture was filtered to remove the catalyst and sodium chloride. The filtrate was evaporated to dryness. 350 ml of THF were added and the mixture was heated to reflux for 30 minutes and then filtered. The filtrate was cooled to 10 °C for crystallization. The obtained solid was collected and dried to give 30 g of N-((S)-1-carbethoxybutyl)-(S)-alanine (HPLC: 89.4%; R-isomer: 2.1%). This solid was again purified with THF as described above to obtain 22 g of the title compound (HPLC: 96.2%; R-isomer: 0.81%; optical rotation: +3.2°).

### Example 6

### Preparation of (2S,3aS,7aS)octahydro-1H-indol-2-carboxylic acid)

(S)-indoline-2-carboxylic acid ethyl ester hydrochloride (43 g), prepared according to Vincent et al., *supra,* and 320 ml of ethanol were hydrogenated in the presence of 4.5 g of 7.5 % Pd/C (dry basis) for 24 h at 60 to 65 °C and at a pressure of 5 MPa. The catalyst was filtered off and 40 ml of an aqueous sodium hydroxide solution (30 %) were added to the filtrate. The mixture was hydrolyzed at 35°C for 5 h. After hydrolysis, the pH was adjusted to 4.5 to 4.8 using 37% aqueous hydrochloric acid. Sodium chloride was removed by filtration and the filtrate was concentrated by distilling off ethanol and water. 160 ml of 1,2-dimethoxyethane and 40 ml of water were added to the dry residue and the mixture was heated to reflux to obtain a clear solution. This solution was cooled for crystallizing. The precipitated solid was collected, dried and purified again using a mixture of 1,2-dimethoxyethan and water as described above to obtain the title compound (assay by titration: 99 %; optical rotation: -44.7°).

### Example 7

### Preparation of (2S,3aS,7aS)octahydro-1H-indol-2-carboxylic acid)

(S)-indoline-2-carboxylic acid (25 g) and 200 ml of methanol were hydrogenated in the presence of 2.5 g of 10 % Pd/C (dry basis) for 40 h at 60 °C and at a pressure of 5 MPa. The catalyst was filtered off and the filtrate was concentrated by distilling off methanol. 50 ml of water were added to the residue and the mixture was heated to 70 °C to obtain a clear solution. Then, at 70 °C 140 ml of 1,2-dimethoxyethane were added dropwise to the clear solution. The mixture turned cloudy and was cooled spontaneously to room temperature. The precipitated solid was collected and dried to obtain 11 g of the title compound (optical rotation: -47.7°). This solid was purified again to obtain 6 g of the title compound (optical rotation: -45.5°).

The same yield and optical rotation can be obtained by using 50 ml of water and 200 ml of THF at 65 °C instead of water and 1,2-dimethoxyethane.

## Claims

1. Process for the preparation of perindopril or of pharmaceutically acceptable salts thereof, wherein
(i) N-((S)-1-carbethoxybutyl)-(S)-alanine or an activated or protected form or a salt thereof
and/or
(ii) (2S,3aS,7aS)-octahydroindole-2-carboxylic acid or an activated or protected form or a salt thereof
is used in a purified form as intermediate, wherein the purified form has been prepared by using
(iii) a solvent selected from the group consisting of tetrahydrofuran, dimethyl carbonate, diethyl carbonate, 1,2-dimethoxy ethane, tert.-butyl methyl ether, 2-methyl tetrahydrofuran, cyclopentyl methyl ether and mixtures thereof,
or a mixture of solvent (iii) and water.

2. Process according to claim 1, wherein a purified form of intermediate (i), preferably N-((S)-1-carbethoxybutyl)-(S)-alanine is used.

3. Process according to claim 2, wherein the purified form has been prepared by a recrystallization from solvent (iii) or a mixture of solvent (iii) and water, preferably from tetrahydrofuran or a mixture of tetrahydrofuran and water.

4. Process according to claim 3, wherein the recrystallization comprises
(a) mixing crude intermediate (i) and solvent (iii) to give a suspension,
(b) optionally adding charcoal to the suspension of step (a),
(c) heating the suspension of step (a) or (b) to obtain a solution of intermediate (i), preferably heating to reflux,
(d) filtering the solution of step (c),
(e) optionally adding water to the filtrate of step (d),
(f) cooling the filtrate of step (d) or the mixture of step (e) to precipitate intermediate (i), and
(g) collecting the obtained intermediate (i).

5. Process according to claim 4, wherein in step (f) the filtrate or mixture is cooled to a temperature of about -20 to about 30 °C.

6. Process according to any one of claims 3 to 5, wherein a mixture of solvent (iii) and water is used and the ratio by volume of solvent (iii) to water ranges from 0.5:1 to 100:1, preferably 1:1 to 50:1, more preferably 5:1 to 30:1.

7. Process according to claim 1, wherein a purified form of intermediate (ii), preferably (2S,3aS,7aS)-octahydroindole-2-carboxylic acid is used.

8. Process according to claim 7, wherein the purified form has been prepared by a recrystallization from solvent (iii) or a mixture of solvent (iii) and water.

9. Process according to claim 8, wherein a mixture of solvent (iii) and water, preferably a mixture of tetrahydrofuran and water or a mixture of 1,2-dimethoxy ethane and water is used.

10. Process according to claim 9, wherein the ratio by volume of solvent (iii) to water ranges from 0.5:1 to 50:1, preferably 1:1 to 20:1, more preferably 2:1 to 8:1.

11. Process according to claim 9 or 10, wherein the recrystallization comprises
(a) heating a mixture of crude intermediate (ii), solvent (iii) and water to give a solution, preferably heating to reflux,
(b) optionally filtering the solution of step (a),
(c) cooling the solution of step (a) or the filtrate of step (b) to precipitate purified intermediate (ii), and
(d) collecting the obtained intermediate (ii).

12. Process according to claim 9 or 10, wherein the recrystallization comprises
(a') heating a mixture of crude intermediate (ii) and water to give a solution, preferably heating to a temperature from 60 to 75 °C,
(b') adding solvent (iii) to the solution of step (a),
(c') cooling the solution of step (b) to precipitate purified intermediate (ii), and
(d') collecting the obtained intermediate (ii).

13. Process according to claim 11 or 12, wherein in step (c) or (c') the solution or filtrate is cooled to a temperature of about -20 to about 30 °C.

14. Process according to any one of claims 11 to 13, wherein prior to step (d) or (d') the solution is kept at the precipitation temperature of step (c) or (c') for 0.5 to 6 h, preferably for 1 to 3 h.

15. Process according to any one of claims 8 to 14, wherein the purified intermediate (ii) is further washed with solvent (iii) or a mixture of solvent (iii) and water.

16. Process for preparing intermediate (i) selected from N-((S)-1-carbethoxybutyl)-(S)-alanine or an activated or protected form or a salt thereof in a purified form, **characterized in that** crude intermediate (i) is purified by using solvent (iii) as defined in claim 1 or a mixture of solvent (iii) and water.

17. Process according to claim 16, wherein the purified form has been prepared by a recrystallization from solvent (iii) or a mixture of solvent (iii) and water.

18. Process for preparing intermediate (ii) selected from (2S,3aS,7aS)-octahydroindole-2-carboxylic acid or an activated or protected form or a salt thereof in a purified form, **characterized in that** crude intermediate (ii) is purified by using solvent (iii) as defined in claim 1 or a mixture of solvent (iii) and water.

19. Process according to claim 18, wherein the purified form is prepared by a recrystallization from solvent (iii) or a mixture of solvent (iii) and water, preferably from a mixture of tetrahydrofuran and water or a mixture of 1,2-dimethoxy ethane and water.
